# EUROPÄISCHE PATENTSCHRIFT

(11) **EP 2 880 147 B1**
(45) Veröffentlichungstag und Bekanntmachung des Hinweises auf die Patenterteilung: **15.06.2016**
(21) Anmeldenummer: 13728680.3
(22) Anmeldetag: 10.06.2013
(51) Int. Cl.: C12M 1/00, C12N 1/06, G01N 1/40

(54) **VORRICHTUNG UND VERFAHREN ZUR BEHANDLUNG EINES FILTRATIONSMEDIUMS**
DEVICE AND METHOD FOR TREATING A FILTRATION MEDIUM
DISPOSITIF ET PROCÉDÉ DE TRAITEMENT D'UN MILIEU DE FILTRATION

(30) Priorität: 31.07.2012 DE 102012015063
(43) Veröffentlichungstag der Anmeldung: 10.06.2015
(73) Patentinhaber: Sartorius Stedim Biotech GmbH, 37079 Göttingen (DE)
(72) Erfinder: PFLANZ, Karl, 37130 Gleichen (DE); SCHOLZ, Alexandra, 37073 Göttingen (DE)
(74) Vertreter: Schneider, Peter Christian
(86) Internationale Anmeldenummer: PCT/EP2013/001691
(87) Internationale Veröffentlichungsnummer: WO 2014/019634

(56) Entgegenhaltungen:
- WO-A2-02/22866
- WO-A2-2011/057707
- DE-A1- 10 054 632

## Beschreibung

Die Erfindung betrifft eine Vorrichtung zur Behandlung eines porösen Filtrationsmediums mit einer aus einem Aufnahmeteil und einem Bodenteil bestehenden Aufnahmeeinheit, wobei mit dem Aufnahmeteil das poröse Filtrationsmedium von einem Unterteil einer Filtrationseinrichtung aufnehmbar und das Aufnahmeteil mit dem porösen Filtrationsmedium auf das Bodenteil aufsetzbar ist, und wobei das Aufnahmeteil mit dem Bodenteil verrastbar ausgebildet ist, wobei das Bodenteil zum Filtrationsmedium hin einen Inkubationsraum aufweist, der mit einem dem Aufnahmeteil abgewandten Auslass des Bodenteils verbunden ist.

Weiterhin betrifft die Erfindung ein Verfahren zur Behandlung eines porösen Filtrationsmediums mit einer aus einem Aufnahmeteil und einem Bodenteil bestehenden Aufnahmeeinheit einer Vorrichtung,
- bei dem das Aufnahmeteil auf das in einem Unterteil einer Filtrationsvorrichtung angeordnete und einer flüssigen Probe ausgesetzte Filtrationsmedium aufgesetzt wird, wobei ein in dem Aufnahmeteil angeordneter Fixierrand mit einem Rand des Filtrationsmediums verbunden wird,
- bei dem das Aufnahmeteil mit dem verbundenen Filtrationsmedium von dem Unterteil abgehoben und auf das Bodenteil aufgesetzt wird, wobei das Aufnahmeteil und das Bodenteil miteinander verrastet werden.

Derzeit werden für Routineuntersuchungen hauptsächlich mikrobiologische Methoden angewandt, die einzelne Mikroorganismen mithilfe von Kultivierungsschritten nachweisen. Diese Methoden sind jedoch sehr zeitaufwändig und können mehrere Tage dauern, bis eine Kontamination des wässrigen Mediums nachgewiesen werden kann. Moderne und schnelle Nachweismethoden für Mikroorganismen, wie Realtime PCR, Antikörper-Assays oder analytische Mikroarrays, ermöglichen eine schnelle Detektion von mikrobieller Kontamination. Um jedoch die Nachweisgrenzen für diese Detektionsverfahren zu senken und idealerweise auch einen einzelnen Keim ausfindig zu machen, sind zuvor schnelle und effektive Anreicherungsschritte notwendig, die ein großes Probevolumen von bis zu mehreren Litern auf wenige hundert Mikroliter einengen. Die aufkonzentrierte Probe bietet ein besseres Handling mit geringerem Verbrauch an Reagenzien und kann beliebig entsprechend der darauffolgenden Detektionsmethode prozessiert werden.

In der Analytik von Flüssigkeiten und Gasen haben sich verschiedene Behandlungsmethoden etabliert, die poröse Medien, wie Filter und Membranen, einsetzen. Zum Beispiel hat sich das Filtrationsverfahren zur Abreicherung und Konzentrierung von gelösten oder partikulären Inhaltsstoffen etabliert. Diese Konzentrierung ist meist notwendig, da die Konzentrationen der Verunreinigungen zu gering sind, um direkte Auswertungen durchzuführen. Die Filtrationsmethoden dienen als Vorstufe für weitere analytische Methoden, wie optische Auswertungen, sowie für weitere physikalische und chemische Reaktionen zur Signalverstärkung.

Für neuere, sensitivere Analysemethoden, zum Beispiel die Polymerasekettenreaktion (PCR), sowie deren Probenvorbereitung können nur geringe Probevolumina verwendet werden. Um die in vielen Fällen typischen Probevolumina von mehr als 100 ml für eine Konzentrierung filtrieren zu können, werden typischerweise Filtrationsmembranen mit 47 mm oder 25 mm Durchmesser eingesetzt. Auch nach der Filtration, wenn die Inhaltsstoffe oder Partikel auf der Filtrationsmembran konzentriert vorliegen, können die membrangebundenen Partikel aufgrund der Membrangröße nicht direkt der Analytik zugeführt werden. Es ist notwendig, die zurückgehaltenen Inhaltsstoffe in ein Probevolumen zu überführen, das idealerweise 1 ml nicht überschreiten sollte, um eine Probenvorbereitung für die anschließende Analytik in Standardreaktionsgefäßen zu ermöglichen, die in Tischzentrifugen passen, die üblicherweise zum Standardequipment eines jeden Labors gehören.

Aus der WO 2011/057707 A2 ist eine Vorrichtung und ein Verfahren zur Behandlung eines porösen Filtrationsmediums mit einer aus einem Aufnahmeteil und einem Bodenteil bestehenden Aufnahmeeinheit bekannt. Mit dem Aufnahmeteil ist das poröse Filtrationsmedium von einem Unterteil einer Filtrationseinrichtung aufnehmbar und das Aufnahmeteil mit dem porösen Filtrationsmedium ist auf das Bodenteil aufsetzbar, wobei das Aufnahmeteil und das Bodenteil miteinander reversibel verbindbar ausgebildet sind. Die bekannte Vorrichtung, die sich grundsätzlich bewährt hat, dient zur Überführung von filtrierten Inhaltsstoffen aus einem Filter (Filtrationsmedium) durch Rückspülung in ein Auffanggefäß, das mit dem Aufnahmeteil verbunden ist.

Nachteilig dabei ist, dass aufgrund der Porengrößenverteilung der meisten Membranfilter zahlreiche Partikel nicht auf der Oberfläche der Membranfilter abgeschieden werden, sondern in tieferen Schichten, so dass keine quantitativ vollständige Rückspülung der Partikel möglich ist. Unspezifische Adsorptionsereignisse der zurückgehaltenen Partikel an der Membran verstärken dieses Problem zusätzlich.

Aus der DE 10 2008 005 968 A1 ist eine Nährmedieneinheit und ein Verfahren zur Aufnahme eines Filters aus einer Filtrationsvorrichtung bekannt. Die Nährmedieneinheit besteht dabei aus einem Deckel bzw. einem Aufnahmeteil, das die eigentliche Transfereinheit bildet, und einem mit Nährmedium gefüllten Unterteil. Das als Aufnahmeteil ausgebildete Oberteil weist dabei einen Fixierrand auf, der zur Entnahme des Filtrationsmediums aus der Filtrations- oder Behandlungsvorrichtung mit einem Rand des Filters über eine Klebehaftung verbindbar ist.

Aus der DE 10 2008 005 968 A1 ist weiterhin ein Verfahren zur mikrobiologischen Untersuchung flüssiger Proben bekannt, bei dem ein Deckel bzw. Aufnahmeteil einer Nährmedieneinheit auf einen in einem Unterteil einer Filter- bzw. Behandlungsvorrichtung angeordneten, als Membranfilter ausgebildeten Filter mit einem Fixierrand aufgesetzt wird. Dabei wird der Fixierrand des Aufnahmeteils über eine Haftschicht mit einem Rand des Filters verbunden. Anschließend wird das Aufnahmeteil mit dem Filter von einer Filterunterstützung des Unterteils der Filtervorrichtung abgehoben und auf einer Oberfläche eines in dem Unterteil einer Nährmedieneinheit angeordneten Nährbodens abgelegt, wobei der Deckel bzw. das Aufnahmeteil das schalenförmig ausgebildete Unterteil abdeckt.

Nachteilig bei den bekannten Filtrationseinheiten und den korrespondierenden Verfahren, die sich für klassische, mikrobiologische Membrananwendungen bewährt haben, in denen man lediglich Partikel entfernt oder im Bereich der Mikrobiologie entstandene Kolonien visuell auswertet, ist aber, dass nach der Filtration die zurückgehaltenen Partikel oder deren Inhaltsstoffe von der Membran nicht mehr in der Weise durch Abspülen zu entfernen sind, dass hochkonzentrierte Suspensionen entstehen.

Ein Auflösen eines porösen Filtrationsmediums mit dem Ziel einer PCR-Analyse der Inhaltsstoffe ist aus der JP 2012-019723 A, aus L. J. DiMichele (Am. Soc. Brew. Chem., 1993, vol.51, No.2, S. 63-66), aus K. Nakamura (Journal of Aerosol Research, 2003, Vol. 18, No.3, S. 177-180) und aus K. Stärk (Applied and Environmental Microbiology, 1998, Vol.64, No.2, S. 543-548) bekannt.

Nachteilig ist jedoch bei jedem dieser Dokumente ein hohes Kontaminationsrisiko, da das Filtrationsmedium mit einer Pinzette aufgenommen, gefaltet und in ein Reaktionsgefäß (meist 1,5 bis 2 ml Gefäß) überführt werden muss. Kontaminationsgefahr besteht ebenfalls durch den nachfolgenden Zusatz des Solvens für die Membran in einem offenen System.

Aus JP 2012-019723 A ist weiterhin bekannt, Cellulosemembranen, auf welchen Mikroorganismen fixiert sind, in Aceton aufzulösen und wässrige Pufferlösungen zuzusetzen, um eine Lösung zu erhalten, welche die Mikroorganismen enthält.

Nachteilig bei diesem Verfahren ist, dass bei diesem Prozess Cellulose in Faserform teilweise wieder ausgefällt wird, wobei die Cellulosefasern in unerwünschter Weise Anteile der Mikroorganismen bzw. der DNA binden und so die quantitative Analyse der Mikroorganismen erschwert wird. Um die unerwünschte Adsorption der Mikroorganismen bzw. DNA auf den Fasern zu verringern, werden bestimmte Additive, z.B. Cetyltrimethylammoniumbromid, zugegeben. Aber eine vollständig quantitative Analyse kann nicht erreicht werden.

Aus L. J. DiMichele (Am. Soc. Brew. Chem., 1993, vol.51, No.2, S. 63-66) ist weiterhin bekannt, Polycarbonatmembranen, auf welchen Mikroorganismen fixiert sind, in einem Gemisch aus Wasser und Chloroform zu lösen (200 µl Wasser und 300 µl Chloroform), mit dem Ziel der Anreicherung der Mikroorganismen in der wässrigen Phase. Nach Aufnahme der wässrigen Phase in ein neues Gefäß erfolgt das Pelletieren der Mikroorganismen mittels Zentrifugation. Es folgen ein Waschschritt und anschließend die PCR.

Nachteilig bei diesem Verfahren ist, dass sich Mikroorganismen in der Praxis nicht in der oberen wässrigen Phase anreichern. Es kommt vielmehr zur Sedimentation der Mikroorganismen in die organische Phase (untere Phase) bzw. in die Grenzschicht, so dass mit dieser Methode keine vollständige Wiederfindung der Mikroorganismen erzielt werden kann. Des Weiteren enthält das beschriebene Verfahren keinen Lyseschritt, um die Mikroorganismen aufzuschließen, so dass davon ausgegangen werden muss, dass zahlreiche intakte Zellen für die PCR eingesetzt werden und somit die Amplifikation für den Großteil der DNA nicht möglich ist.

Aus K. Stärk (Applied and Environmental Microbiology, 1998, Vol.64, No.2, S. 543-548) ist weiterhin bekannt, Polyethersulfonmembranen, auf welchen Mikroorganismen fixiert sind, in Chloroform zu lösen. Anschließend erfolgen die Zugabe von TE-Puffer und eine zehnminütige Extraktion der DNA in die wässrige Phase unter Schütteln bei Raumtemperatur. Die wässrige Lösung soll anschließend einer alkoholischen DNA-Fällung unterzogen werden, bevor die Auswertung mittels PCR geschieht.

Nachteilig bei diesem Verfahren ist, dass nur ein geringer Anteil der DNA in die wässrige Phase extrahiert werden kann, da zuvor kein Lyseschritt erfolgt ist, um die Mikroorganismen aufzuschließen und somit die DNA frei zugänglich zu machen. Unter Verwendung dieser Methode kommt es vielmehr zur Sedimentation der noch intakten Mikroorganismen in die untere organische Phase bzw. in die Grenzschicht zwischen organischer und wässeriger Phase.

Auch aus K. Sen (Applied and Environmental Microbiology, 2007, vol. 73, No. 22, S. 7380-7387) ist bekannt, das Filtrationsmedium mit einer Pinzette zu falten und in ein Reaktionsgefäß zu überführen. Es folgt kein Auflöseschritt der Membran, sondern lediglich ein Abspülschritt mit beispielsweise einem kommerziellen Lysepuffer oder eine mechanische Beanspruchung der Membran durch Vortexen in Anwesenheit von Mahlkugeln, was dem Zellaufschluss dient. Bei K. Sen kommen verschiedene kommerzielle Kits zur DNA-Isolierung zum Einsatz.

Nachteilig bei den beschriebenen Verfahren ist nicht nur die erhöhte Kontaminationsgefahr durch das Falten und Überführen der Membran mithilfe einer Pinzette, sondern auch, dass ein vollständiges Abspülen der Mikroorganismen von der Membran nicht möglich ist, da Mikroorganismen häufig auch in tieferen Schichten der Membran abgeschieden werden und es zusätzlich zu unspezifischer Adsorption an die Membran kommen kann, so dass ein oberflächlicher Spülschritt nicht effektiv ist. Erschwerend kommt hinzu, dass bei einer klein zusammengefalteten Membran in einem Reaktionsgefäß keine gezielte reverse Spülung der Membran erfolgen kann, lediglich ein ungerichtetes Mixen bzw. Vortexen von Membran und Spüllösung kann erreicht werden.

Aus der WO 2012/031156 A1 ist eine Filtrationsvorrichtung bekannt, die ein kontaminationsfreies Arbeiten dadurch ermöglicht, dass das Filtrationsmedium (13 mm Durchmesser der Filtrationsfläche) in der verschließbaren Vorrichtung verbleibt und der Zellaufschluss direkt auf der Membran mithilfe von Mahlkugeln und Vortexen erfolgt. Die freie DNA passiert in einem anschließenden Filtrationsschritt die Membran.

Nachteilig bei diesem Verfahren ist, dass kein quantitativ vollständiger Zellaufschluss möglich ist, da meist ein Großteil der Mikroorganismen in tiefere Membranschichten eindringt und somit vor den Mahlkugeln abgeschirmt wird. Des Weiteren hat diese Pufferwirkung einen negativen Einfluss auf den Aufschlussgrad der Mikroorganismen, da ein Großteil der Stöße von der Membran abgefangen wird. Auch wird der anschließende Filtrationsschritt der DNA durch die Membran nicht vollständig ablaufen, da DNA dazu neigt, unspezifische Bindungen, in diesem Fall an der Membran, einzugehen. Des Weiteren ist der Durchmesser der Filtrationsfläche bei dieser Vorrichtung auf 13 mm limitiert, aufgrund der Kompatibilität zu gängigen Zentrifugenmodellen und -adaptoren. Dieser geringe Durchmesser des Filtrationsmediums führt jedoch bei größeren Probevolumina zu deutlich längeren Filtrationszeiten.

Die EP 2 402 456 A1 offenbart ein Verfahren zur Analyse von Mikroorganismen in Wasserproben, bei welchem mittels einer ersten Spritze eine Mikroorganismen enthaltende Wasserprobe in einen Minisart^{®}-Spritzenvorsatzfilter mit einer Celluloseestermembran zur Retention der Mikroorganismen gespritzt wird. Nach Entfernung der Spritze wird das eine Ende des Spritzenvorsatzfilters mit einem Auffanggefäß verbunden, während das zweite Ende des Spritzenvorsatzfilters mit einer zweiten, mit einem polar aprotischen Lösungsmittel, wie DMSO (Dimethylsulfoxid), gefüllten Spritze verbunden wird. DMSO wird bis zum Erreichen des Druckpunkts in den Spritzenvorsatzfilter gespritzt, um die Membran mit den zurückgehaltenen Mikroorganismen aufzulösen und die Lösung in dem Auffanggefäß aufzufangen. An die Zentrifugation der Lösung in dem Auffanggefäß schließen sich eine Zelllyse und weitere mikrobiologische Analyseschritte, wie eine PCR, an.

Nachteilig bei dem aus EP 2 402 465 A1 bekannten Verfahren sind das sukzessive Hantieren mit zwei verschiedenen Spritzen, von denen eine die Wasserprobe und die andere das Lösungsmittel für die Celluloseestermembran enthält, und die Tatsache, dass beim Einspritzen von DMSO in den Spritzenvorsatzfilter der Einspritzdruck nicht zu hoch, d.h. unterhalb des Druckpunkts, sein darf, damit der Spritzenvorsatzfilter nicht beschädigt wird.

Weiter ist aus der WO 02/22866 A2 ein Verfahren zur Analyse von nucleinsäurehaltigem Material bekannt. Das bekannte Verfahren umfasst das Abscheiden des nucleinsäurehaltigen Materials auf einem Filtermaterial und das Auflösen des Filtermaterials in einem Lösungsmittel, wobei das nucleinsäurehaltige Material auf dem Filtermaterial aufgeschlossen wird.

Aufgabe der vorliegenden Erfindung ist es daher, eine Vorrichtung und ein Verfahren bereitzustellen, bei dem es möglich ist, ein poröses Filtrationsmedium einschließlich zurückgehaltener Mikroorganismen kontaminationsfrei, einfach und sicher in ein Auffanggefäß zu überführen, um die Probe quantitativ vollständig für eine DNA-Extraktion und molekularbiologische Analysemethoden zugänglich zu machen.

Die die Vorrichtung betreffende Aufgabe wird in Verbindung mit dem Oberbegriff des Anspruches 1 dadurch gelöst, dass der Inkubationsraum des Bodenteiles zum Auslass hin konisch verläuft, dass der Auslass einen Ansatz aufweist, auf den ein ein Lösungsmittel zum Auflösen des porösen Filtrationsmediums enthaltendes Auffanggefäß lösbar aufsteckbar ist, dass das auf das Bodenteil aufzusteckende Auffanggefäß neben dem Lösungsmittel den Zellaufschluss unterstützende Mahlkugeln enthält, und dass der Auslass des Bodenteils einen Auslasskanal aufweist, der quer zur Längsachse des Bodenteils als länglicher Schlitz ausgebildet ist, dessen schmale lichte Weite kleiner ist als die Außendurchmesser der Mahlkugeln.

Durch die Anordnung des Inkubationsraumes im Bodenteil und die Verbindung über den Auslass zum Auffanggefäß mit dem Lösungsmittel ist es möglich, das poröse Filtrationsmedium einschließlich zurückgehaltener Mikroorganismen köntaminationsfrei, einfach und sicher in das Auffanggefäß zu überführen, um die Probe quantitativ vollständig für eine DNA-Extraktion und molekularbiologische Analysemethoden zugänglich zu machen.

Der Inkubationsraum des Bodenteiles verläuft zum Auslass hin konisch. Die konische Form garantiert, dass trotz des Zentrifugationswinkels einer Festrotorzentrifuge kein Totvolumen im Inkubationsraum der Aufnahmeeinheit entsteht, was zu Restflüssigkeit führen könnte. Ohne die konische Form würde wegen der Zentrifugalkraft, abhängig vom Zentrifugenmodell, Restflüssigkeit seitlich im Bodenteil der Aufnahmeeinheit verbleiben. Die Anordnung von Mahlkugeln fördert den Zellaufschluss.

Nach einer bevorzugten Ausführungsform der Erfindung ist das Lösungsmittel zum Auflösen des Filtrationsmediums ein organisches Lösungsmittel, bevorzugt Chloroform oder Methylenchlorid.

Gemäß einer bevorzugten Ausführungsform der Erfindung enthält das auf das Bodenteil aufzusteckende Auffanggefäß neben dem Lösungsmittel den Zellaufschluss unterstützende Mahlkugeln.

Gemäß einer bevorzugten Ausführungsform der Erfindung enthält das auf das Bodenteil aufzusteckende Auffanggefäß neben dem Lösungsmittel einen den Zellaufschluss unterstützenden Lysepuffer. Bei dem Lysepuffer handelt es sich bevorzugt um eine wässrige Flüssigkeit, welche nicht mit dem Lösungsmittel emulgiert, sondern mit dem Lösungsmittel ein Zweiphasensystem bildet.

Gemäß einer weiteren bevorzugten Ausführungsform der Erfindung enthält das auf das Bodenteil aufzusteckende Auffanggefäß neben dem Lösungsmittel einen den Zellaufschluss unterstützenden Lysepuffer.

Das Auffanggefäß ist an seinem offenen Ende durch einen Deckel flüssigkeitsdicht verschließbar. Dazu weist das Auffanggefäß beispielsweise an seinem offenen Ende ein Außengewinde auf, mit dem der Deckel verschraubt werden kann.

Nach einer weiteren bevorzugten Ausführungsform der Erfindung weist der Auslass des Bodenteils einen Auslasskanal auf, der quer zur Längsachse des Bodenteils als länglicher Schlitz ausgebildet ist, dessen schmale lichte Weite kleiner ist als die Außendurchmesser der Mahlkugeln. Dadurch wird sichergestellt, dass die Mahlkugeln nicht in den Inkubationsraum eindringen und im Auffanggefäß verbleiben.

Gemäß einer weiteren bevorzugten Ausführungsform der Erfindung ist eine Innenwandung des Aufnahmeteils außerhalb einer für eine Filtration nutzbaren Fläche des Filtrationsmediums positionierbar und ein in dem Aufnahmeteil angeordneter Fixierrand ist auf einem Rand des Filtrationsmediums positionierbar, und der Fixierrand des Aufnahmeteils ist mit dem Rand des Filtrationsmediums über eine Klebehaftung verbindbar. Bei einer alternativen Ausführungsform der Erfindung ist ein Fixierrand des Aufnahmeteils durch eine mechanische Klemmverbindung mit einem korrespondierenden ringförmigen Klemmteil am Rand des Filtrationsmediums verbindbar. Damit lässt sich das Aufnahmeteil leicht mit dem Filtrationsmedium verbinden und Aufnahmeteil und Filtrationsmedium lassen sich einfach und kontaminationsfrei auf das Bodenteil aufsetzen. Das Filtrationsmedium ist bevorzugt aus Polycarbonat oder Polyethersulfon ausgebildet.

Nach einer weiteren bevorzugten Ausführungsform der Erfindung ist die Aufnahmeeinheit mit dem in vertikaler Richtung unten angeordneten Auffanggefäß in einem Zentrifugenadapter befestigbar und kann mit dem Zentrifugenadapter in einer Zentrifuge zentrifugiert werden, wobei das in dem Lösungsmittel gelöste Filtrationsmedium einschließlich zurückgehaltener Mikroorganismen vollständig in das Auffanggefäß überführbar ist.

Die Aufgabe betreffend das Verfahren wird in Verbindung mit dem Oberbegriff des Anspruches 9 dadurch gelöst, dass ein ein Lösungsmittel zum Auflösen des porösen Filtrationsmediums und den Zellaufschluss unterstützende Mahlkugeln enthaltendes Auffanggefäß mit einem am Bodenteil angeordneten Auslass lösbar verbunden wird, und dass die Aufnahmeeinheit mit dem Auffanggefäß kopfüber geschüttelt wird, wobei das Lösungsmittel über den Auslass des Bodenteils dem Filtrationsmedium zugeführt wird und das Filtrationsmedium auflöst, und dass die Aufnahmeeinheit mit dem in vertikaler Richtung unten angeordneten Auffanggefäß in einem Zentrifugenadapter befestigt und in einer Zentrifuge zentrifugiert wird, wobei das in dem Lösungsmittel gelöste Filtrationsmedium einschließlich zurückgehaltener Mikroorganismen vollständig in das Auffanggefäß überführt wird.

Das Filtrationsmedium kann einfach mit dem Aufnahmeteil der Aufnahmeeinheit von einem Unterteile einer Filtrationsvorrichtung abgenommen und auf das Bodenteil aufgesetzt werden. Nach Entfernen eines Deckels von dem Auffanggefäß mit Lösungsmittel lässt sich das Auffanggefäß einfach und kontaminationsfrei auf einen Auslass des Bodenteils aufstecken. Durch ein leichtes Schütteln der kopfüber gewendeten Aufnahmeeinheit wird das Lösungsmittel dem Inkubationsraum zugeführt und das Filtrationsmedium innerhalb weniger Sekunden aufgelöst und anschließend in das Auffanggefäß überführt. Obwohl im Inkubationsraum der Aufnahmeeinheit ein erhöhter Druck aufgrund des teilweise verdampfenden organischen Lösungsmittels entsteht, bleiben das Aufnahmeteil und das Bodenteil aufgrund ihrer Verrastung miteinander verbunden.

Das aufgelöste Filtrationsmedium mit den zurückgehaltenen Partikeln bietet einen guten Ausgangspunkt für eine weitere Probenvorbereitung, z.B. eine DNA-Extraktion, und für verschiedene analytische Methoden, wie beispielsweise PCR.

Die dem Auffanggefäß neben dem Lösungsmittel zugeführten Mahlkugeln fördern den Zellaufschluss.

Die Aufnahmeeinheit wird mit dem in vertikaler Richtung unten angeordneten Auffanggefäß in einem Zentrifugenadapter befestigt und in einer Zentrifuge zentrifugiert, wobei das in dem Lösungsmittel gelöste Filtrationsmedium einschließlich zurückgehaltener Mikroorganismen vollständig in das Auffanggefäß überführt wird.

Im Anschluss wird das Auffanggefäß von der Aufnahmeeinheit entfernt und mit einem Deckel verschlossen.

Gemäß einer weiteren bevorzugten Ausführungsform der Erfindung wird das Auffanggefäß von der Aufnahmeeinheit entfernt und mit einem Deckel verschlossen, wobei vor dem Verschließen mit dem Deckel die Zugabe von einem den Zellaufschluss unterstützenden Lysepuffer in das Auffanggefäß erfolgt.

Die Verwendung des Lysepuffers kann zusätzlich zur Verwendung von Mahlkugeln erfolgen. Bei dem Lysepuffer handelt es sich bevorzugt um eine wässrige Flüssigkeit, die nicht mit dem Lösungsmittel emulgiert, sondern zur Ausbildung von zwei getrennten Phasen (Lösungsmittel und Lysepuffer) führt.

Beispielhaft als geeigneter Lysepuffer für das erfindungsgemäße Verfahren sei hier das Produkt "Cell Lysis Solution" (Mat. 2900024) von 5Prime genannt. Das verschlossene Auffanggefäß wird anschließend schüttelnd inkubiert. Für den Fall, dass für den Zellaufschluss sowohl Lysepuffer als auch Mahlkugeln zum Einsatz kommen, erfolgt die den Aufschluss unterstützende Inkubation in einem Homogenisator. Kommt nur Lysepuffer zum Einsatz, kann die Zugabe von Tris-EDTA-Puffer mit 0,01 % Natriumdodecylsulfat (siehe Beispiel 1, Schritt 9) entfallen, da bereits der verwendete Lysepuffer zur Ausbildung zweier Phasen führt und sich die DNA der lysierten Mikroorganismen in der wässrigen Lysepuffer-Phase anreichert.

Gemäß einer weiteren bevorzugten Ausführungsform der Erfindung wird das verschlossene Auffanggefäß in einem Schüttelinkubator oder Homogenisator prozessiert, wobei ein Zellaufschluss der Mikroorganismen mithilfe der Mahlkugeln und/oder des Lysepuffers ermöglicht wird.

Die Vorrichtung zur Behandlung des Filtrationsmediums kann steril verpackt geliefert werden.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

In den Zeichnungen zeigen:

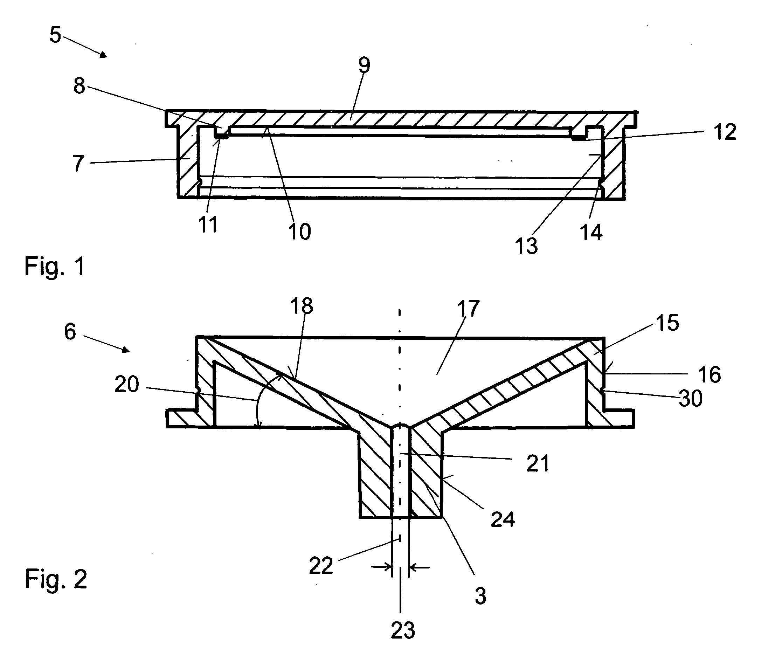

- Figur 1:: eine Seitenansicht im Schnitt eines Aufnahmeteils einer Vorrichtung zur Behandlung eines porösen Filtrationsmediums,
- Figur 2:: eine Seitenansicht im Schnitt eines Bodenteils einer Vorrichtung zur Behandlung eines porösen Filtrationsmediums,
- Figur 3:: eine Untersicht auf das Bodenteil von Fig. 2,
- Figur 4:: eine Seitenansicht im Schnitt einer Aufnahmeeinheit mit aufgenommenem porösen Filtrationsmedium, deren Aufnahmeteil und Bodenteil miteinander verrastet sind, und aufgestecktem Auffangbehälter mit Lösungsmittel und Mahlkugeln,
- Figur 5:: eine vergrößerte Darstellung der Einzelheit V (Verrastung) von Figur 4,
- Figur 6:: eine vergrößerte Darstellung einer weiteren Verrastung entsprechend der Einzelheit V von Figur 4,
- Figur 7:: eine Seitenansicht im Schnitt einer weiteren Aufnahmeeinheit mit aufgenommenem porösen Filtrationsmedium, deren Aufnahmeteil und Bodenteil miteinander verrastet sind, und aufgestecktem Auffangbehälter mit Lösungsmittel und Mahlkugeln,

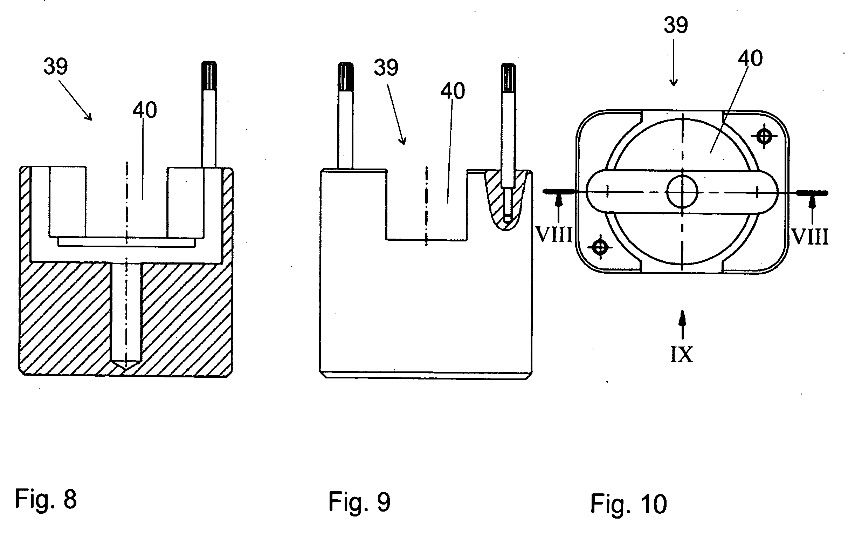

- Figur 8:: eine Seitenansicht im Schnitt des Zentrifugenadapters von Fig.10 entlang der Linie VIII-VIIIgeschnitten,
- Figur 9:: eine Seitenansicht des Zentrifugenadapters von Fig. 10 aus Richtung IX,
- Figur 10:: eine Draufsicht auf den Zentrifugenadapter der Figuren 8 und 9,
- Figur 11:: eine Seitenansicht im Schnitt einer Filtrationsvorrichtung nach dem Stand der Technik mit auf einem Unterteil angeordnetem Filtrationsmedium,
- Figur 12:: Kurvenverläufe der nach einer bevorzugten Ausführungsform der Erfindung prozessierten *B. subtilis* Proben und
- Figur 13:: Kurvenverläufe der nach der bevorzugten Ausführungsform der Erfindung prozessierten *B. subtilis* Sporen-Proben im Vergleich zum Stand der Technik.

### Beschreibung der Ausführungsbeispiele

Eine Vorrichtung 1 besteht im Wesentlichen aus einer Aufnahmeeinheit 2 mit einem Auslass 3 und einem Auffanggefäß 4.

Die Aufnahmeeinheit 2 ist zweiteilig ausgebildet und besteht aus einem Aufnahmeteil 5 und einem Bodenteil 6.

Das Aufnahmeteil 5 bildet eine umlaufende Kontur mit einer Außenwandung 7 und einer parallel verlaufenden Innenwandung 8. Das Aufnahmeteil 5 wird in vertikaler Richtung oben von einer Deckenwandung 9 abgeschlossen. Die Deckenwandung 9 weist auf ihrer dem Bodenteil 6 zugewandten Aufnahmeteilinnenfläche 10 die Innenwandung 8 auf, deren freies Ende mit seiner Stirnfläche einen Fixierrand 11 bildet. In den Ausführungsbeispielen weist der Fixierrand 11 eine Haftschicht 12 aus einem geeigneten Klebestoff auf.

Die Haftschicht 12 ist beispielsweise aus einem PSA-Dispersionsklebstoff oder aus Acrylat-Copolymer-Mikrokugeln ausgebildet. Geeignete Kleber sind Kleber, die auf organischen Lösungsmittel(n) basieren und im organischen Lösungsmittel lösbar sind, welche im Rahmen des Auflöseprozesses eines Filtrationsmediums Anwendung finden. Des Weiteren müssen die Kleber eine permanente Haftkraft zeigen (ab Produktion der Vorrichtung bis zum Einsatz beim Anwender). Der Kleber sollte mittels ETO (Ethylenoxid) sterilisierbar sein. Zudem werden Kleber eingesetzt, die keine unspezifischen Reaktionen oder Signale mit in der nachfolgenden Analytik eingesetzten Reagenzien und Reaktionsmethoden zeigen. Insbesondere ist der Kleber bevorzugt DNA-frei und weist keine Substanzen auf, die durch Färbung, Fluoreszenz oder chemische Reaktion die anschließende Analytik stören.

Die Außenwandung 7 weist eine innere Außenwandungsfläche 13 mit einer umlaufenden Wulst 14 auf.

Das Bodenteil 6 weist eine umlaufende Außenwandung 15 mit einer Außenfläche 16 auf, die mit der Innenwandung 13 des Aufnahmeteils 5 korrespondiert, also zusammenwirkt. Zum Aufnahmeteil 5 hin weist das Bodenteil 6 einen trichterförmigen Inkubationsraum 17 mit einer konischen Abflussfläche 18 auf, die gegenüber einer Horizontalen mit einem zu dem Auslass 3 hin abfallenden Winkel 20 von beispielsweise 2-5° gezeigt ist. Der Auslass 3 weist einen Auslasskanal 21 auf, der quer zur Längsachse 22 des Bodenteils 6 als länglicher Schlitz mit einer schmalen lichten Weite 23 ausgebildet ist.

Die außenliegende Mantelfläche des Auslasses 3 bildet einen leicht konischen Absatz 24, auf den das Auffanggefäß 4 lösbar aufsteckbar ist. An seinem offenen Ende 26 weist das Auffanggefäß 4 ein Außengewinde 27 auf und ist mit einem nicht dargestellten Deckel mit Innengewinde durch Verschrauben dicht verschließbar. In dem Auffanggefäß 4 sind ein Lösungsmittel 28 und Mahlkugeln 29 vorgelegt.

Alternativ kann in dem Auffanggefäß 4 statt oder zusätzlich zu den Mahlkugeln 29 ein Lysepuffer für die von einem Filtrationsmedium 37 zurückgehaltenen Mikroorganismen vorgelegt werden, wobei der Lysepuffer als wässrige Flüssigkeit mit dem Lösungsmittel 28 ein Zweiphasensystem ausbildet.

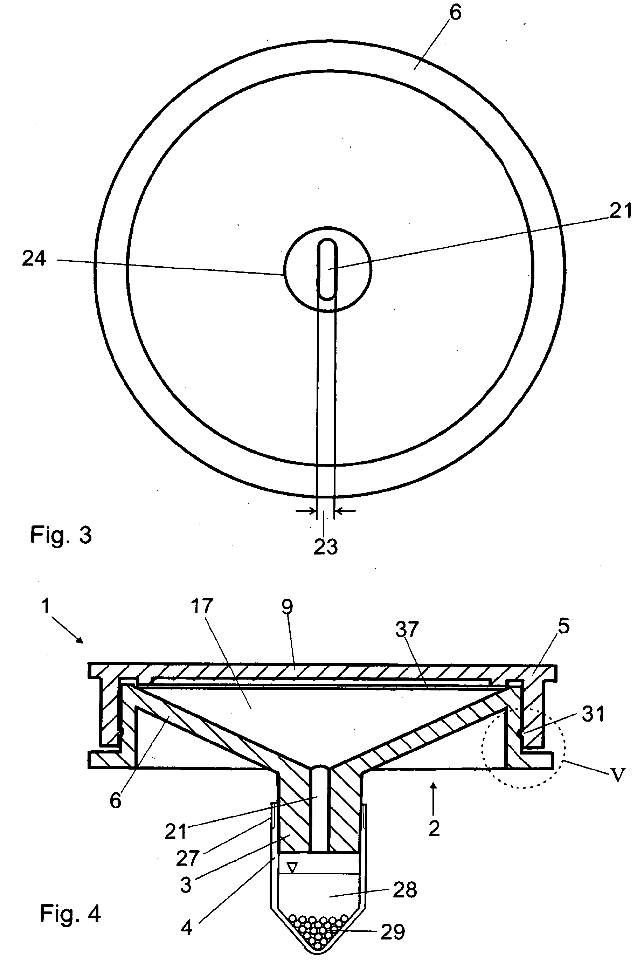

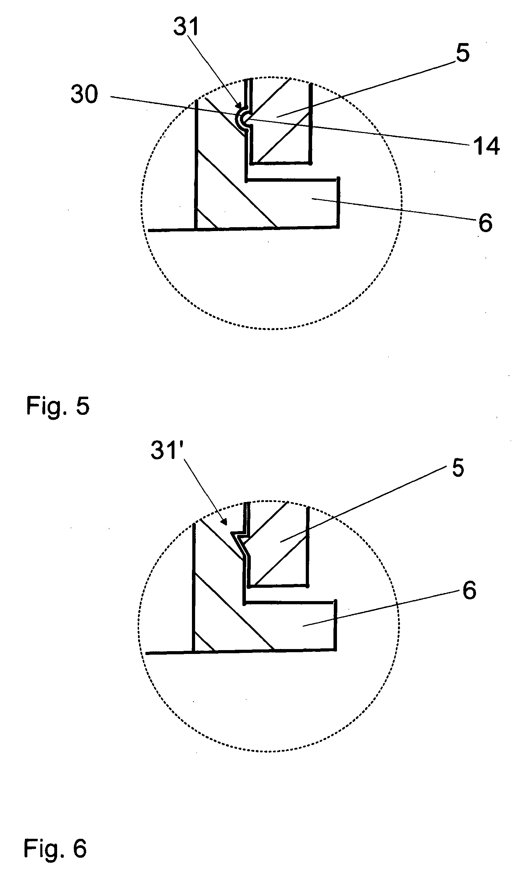

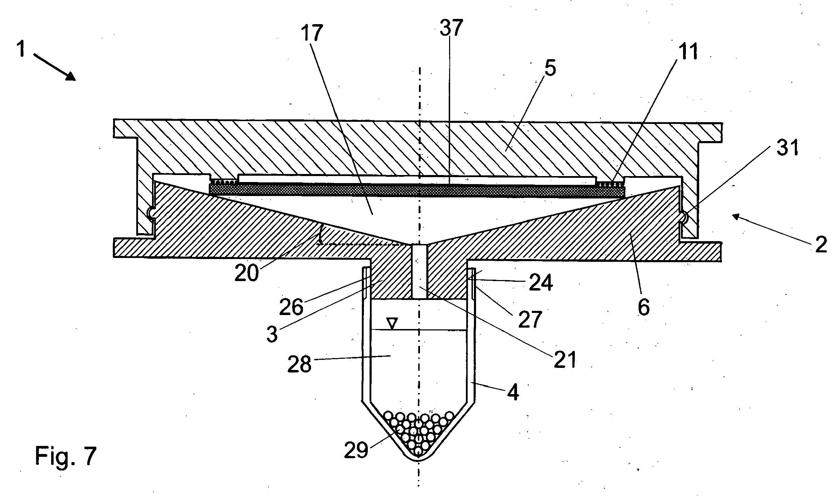

Das Bodenteil 6 weist an seiner Außenfläche 16 eine umlaufende ringförmige Vertiefung 30 auf, die mit der Wulst 14 des Aufnahmeteils 5 korrespondiert und eine Verrastung 31 bildet (s. Fig. 4, 5, 7). Die Verrastung 31 kann auch entsprechend Fig. 6 irreversibel ausgebildet sein.

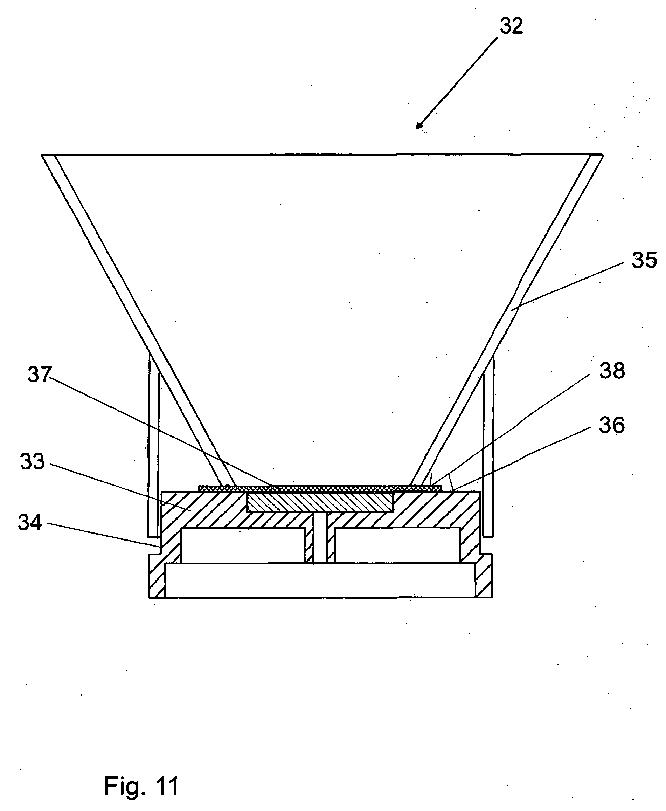

Eine an sich bekannte Filtrationsvorrichtung 32 entsprechend Figur 11 besteht aus einem Unterteil 33 mit einem Aufnahmeabsatz 34, auf dem ein trichterförmiger Aufsatz 35 aufsetzbar ist. Zwischen dem Aufsatz 35 und einer Filterunterstützungsfläche 36 des Unterteils 33 ist das bevorzugt scheibenförmige Filtrationsmedium 37, das beispielsweise als eine poröse Filtermembran ausgebildet ist, angeordnet.

Nach einem Filtrationsvorgang kann der Aufsatz 35 von dem Unterteil 33 abgenommen werden und das Aufnahmeteil 5 der Vorrichtung 1 kann anstelle des Aufsatzes 35 auf das Unterteil 33 aufgesetzt werden. Dabei wird das Aufnahmeteil 5 mit seinem Fixierrand 11 auf einen Rand 38 des scheibenförmigen Filtrationsmediums 37 aufgesetzt, so dass das scheibenförmige Filtrationsmedium 37 an der Haftschicht 12 des Fixierrandes 11 haftet und von dem Unterteil 33 aufgenommen werden kann.

Die Vorrichtung 1 kann mit dem Auffanggefäß 4 in einen Zentrifugenadapter 39 eingesetzt werden, der eine entsprechend angepasste Aussparung 40 aufweist.

Zur Behandlung des porösen Filtrationsmediums 37 mit der aus dem Aufnahmeteil 5 und dem Bodenteil 6 bestehenden Aufnahmeeinheit 2 der Vorrichtung 1 werden die folgenden Schritte durchgeführt:
- das Aufnahmeteil 5 wird auf das in dem Unterteil 33 der Filtrationsvorrichtung 32 angeordnete und einer flüssigen Probe ausgesetzte Filtrationsmedium 37 aufgesetzt, wobei ein in dem Aufnahmeteil 5 angeordneter Fixierrand 11 mit einem Rand 38 des Filtrationsmediums 37 verbunden wird,
- das Aufnahmeteil 5 mit dem verbundenen Filtrationsmedium 37 wird von dem Unterteil 33 abgehoben und auf das Bodenteil 6 aufgesetzt, wobei das Aufnahmeteil 5 und das Bodenteil 6 miteinander über die Verrastung 31, 31' verrastet werden,
- ein Lösungsmittel 28 zum Auflösen des porösen Filtrationsmediums 37 und Mahlkugeln 29 enthaltendes Auffanggefäß 4 wird mit dem am Bodenteil 6 angeordneten Auslass 3 lösbar verbunden,
- die Aufnahmeeinheit 2 mit dem Auffanggefäß 4 wird kopfüber leicht geschüttelt, wobei das Lösungsmittel 28 über den Auslass 3 des Bodenteils 6 dem Filtrationsmedium 37 zugeführt wird und das Filtrationsmedium 37 auflöst.

Danach können die nachfolgenden Schritte durchgeführt werden:
- die Aufnahmeeinheit 2 mit dem in vertikaler Richtung unten angeordneten Auffanggefäß 4 wird in dem Zentrifugenadapter 39 befestigt und in einer Zentrifuge zentrifugiert, wobei das in dem Lösungsmittel 28 gelöste Filtrationsmedium 37 einschließlich zurückgehaltener Mikroorganismen vollständig in das Auffanggefäß 4 überführt wird,
- das Auffanggefäß 4 wird von der Aufnahmeeinheit 2 entfernt und mit einem Deckel durch Verschrauben verschlossen,
- das verschlossene Auffanggefäß 4 wird in einem Homogenisator prozessiert, wobei ein Zellaufschluss der Mikroorganismen mithilfe der Mahlkugeln 29 ermöglicht wird.

Sofern an Stelle von oder zusätzlich zu den Mählkugeln 29 für den Zellaufschluss ein Lysepuffer verwendet wird, können die nachfolgenden Schritte durchgeführt werden:
- die Aufnahmeeinheit 2 mit dem in vertikaler Richtung unten angeordneten Auffanggefäß 4 wird in dem Zentrifugenadapter 39 befestigt und in einer Zentrifuge zentrifugiert, wobei das in dem Lösungsmittel 28 gelöste Filtrationsmedium 37 einschließlich zurückgehaltener Mikroorganismen vollständig in das Auffanggefäß 4 überführt wird,
- das Auffanggefäß 4 wird von der Aufnahmeeinheit 2 entfernt und vor dem Verschließen mit einem Schraubdeckel mit dem Lysepuffer gefüllt,
- das verschlossene Auffanggefäß 4 wird in einem Homogenisator prozessiert, wobei ein Zellaufschluss der Mikroorganismen mithilfe der Mahlkugeln 29 und/oder des Lysepuffer ermöglicht wird.

Erfolgt der Zellaufschluss ohne Verwendung von Mahlkugeln nur mit Lysepuffer, kann der Zellaufschluss alternativ auch in einem Schüttelinkubator statt in einem Homogenisator erfolgen.

Es wurden die folgenden Versuche durchgeführt:

### Beispiel 1

Bestimmung der Sensitivität für den Nachweis von *Bacillus subtilis* unter Verwendung der Vorrichtung 1 inklusive Auffanggefäß 4.

Es wurde eine Verdünnungsreihe in 0,9 % NaCl-Lösung einer in der exponentiellen Phase befindlichen *Bacillus subtilis* Kultivierung in Doppelbestimmung nach Filtration auf Sartorius-Nähragar inkubiert (47 mm Cellulosenitratmembran mit 0,45 µm Porendurchmesser; Auszählung der Kolonien nach 24 h) und parallel pro Verdünnungsstufe wurde eine Probe nach einer bevorzugten Ausführungsform der Erfindung prozessiert.

Eine bevorzugte Ausführungsform der Erfindung umfasst folgende Prozessschritte:
1. Membranfiltration einer wässrigen Probe (47 mm Membrandurchmesser, Track-Etched"-Polycarbonatmembran, 0,4 µm Porendurchmesser, 6 bis 11 µm Membrandicke) unter Verwendung eines Unterteils 33 (idealerweise aus Plastik; ETO-steril) wie in Fig. 11 dargestellt.
2. Entfernung des Aufsatzes 35 und Aufnahme des Membranfilters/Filtrationsmediums 37 mithilfe der Klebhaftung am Fixierrand 11 vom Aufnahmeteil 5 (aus Polypropylen; ETO-steril).
3. Verbinden des Aufnahmeteils 5 und Bodenteils 6 der Aufnahmeeinheit 2 mittels Verrastung 31.
4. Auffanggefäß 4 (aus Polypropylen; ETO-steril) mit anhängendem Schraubdeckel mit einem Fassungsvolumen von 2 ml enthält 15 Stahlkugeln/Mahlkugeln 29 (3 mm Durchmesser) und 750 µl Chloroform ("Molecular Biology Grade"; vor allem DNA- und DNase-frei). Nach dem Öffnen des Auffanggefäßes 4 ist dieses am Auslass 3 des Bodenteils 6 über eine Steckverbindung zu befestigen (sich nach unten hin verjüngender Ansatz 24 wird in offenes Ende 26 des Auffanggefäßes 4 gesteckt).
5. Die Aufnahmeeinheit 2 mit Auffanggefäß 4 wird kopfüber leicht geschüttelt, um das Chloroform vollständig vom Auffanggefäß 4 in die Aufnahmeeinheit 2 zu überführen. Anschließend wird die Aufnahmeeinheit 2 mit dem Auffanggefäß 4 kopfüber wenige Sekunden leicht geschwenkt, um ein vollständiges Auflösen des Membranfilters/Filtrationsmediums 37 zu gewährleisten.
6. Die Aufnahmeeinheit 2 mit befestigtem Auffanggefäß 4 wird aufrecht (Aufnahmeteil 5 zeigt nach oben, Auffanggefäß 4 zeigt nach unten) in den speziellen Zentrifugenadapter 39 überführt. Es handelt sich hierbei um einen Ausschwingzentrifugenadapter, um ein quantitativ vollständiges Überführen der gelösten Membran/Filtrationsmedium 37 inklusive der durch die Membran 37 zurückgehaltenen Partikel und des Lösungsmittels 28 zu gewährleisten. Des Weiteren ist der Zentrifugenadapter 39 derart konstruiert, dass ein Abfallen/Lösen des Auffanggefäßes 4 während des Zentrifugationsschrittes unterbunden wird. Der Zentrifugenadapter 39 wird in eine passende Zentrifuge montiert und es folgt eine einminütige Zentrifugation bei mind. 3.000 x g, um die im Chloroform gelöste Membran 37 inkl. zurückgehaltenen Mikroorganismen vollständig in das Auffanggefäß 4 mit den Mahlkugeln 29 aus Stahl zu überführen.
7. Das Auffanggefäß 4 wird von der Aufnahmeeinheit 2 entfernt und mithilfe des anhängenden Schraubdeckels fest verschlossen.
8. Um die Mikroorganismen aufzuschließen und deren DNA zugänglich zu machen, wird das Auffanggefäß 4 2 min bei 6,5 m/s im Homogenisator "FastPrep-24 Instrument" (MP Biomedicals) prozessiert (Alternativ sind auch andere Homogenisatoren mit vergleichbarer Leistung einsetzbar).
9. Das Auffanggefäß 4 wird aus dem Homogenisator entnommen und es werden 500 µl 1x TE-Puffer (Tris-EDTA) mit 0,01 % SDS (Natriumdodecylsulfat) hinzugefügt ("Molecular Biology Grade").
10. Es folgt eine 10-minütige Extraktion (DNA aus organischer in wässrige Phase extrahieren) bei Raumtemperatur. Hierfür ist das Auffanggefäß 4 entweder horizontal auf einem Vortexer oder horizontal auf einem Thermomixer bei 750 rpm zu befestigen.
11. In die Öffnung des Auffanggefäßes 4 eine Spatelspitze DNA- und DNase-freie Silikonpaste geben (z.B. Phase Lock Gel von 5 Prime oder GE Bayer Silicones, hochviskos).
12. Das Auffanggefäß 4 3 min bei 16.000 x g zentrifugieren.
13. Es haben sich drei Phasen aufgrund der unterschiedlichen Dichten separiert: Die obere wässrige Phase inkl. DNA, die mittlere Silikongelphase als Sperrschicht und die untere organische Phase. Die obere wässrige Phase wird mithilfe einer Pipette vollständig in ein neues, leeres Auffanggefäß 4 überführt (ein 1,5 ml Reaktionsgefäß ist ausreichend).
14. 600 µl Isopropanol ("Molecular Biology Grade") und 2 µl Glycogen als DNA-Carrier ("Molecular Biology Grade") hinzufügen und 50-mal das Reaktionsgefäß 4 invertieren.
15. Das Reaktionsgefäß 4 3 min bei 16.000 x g zentrifugieren, so dass ein kleines weißes DNA-Glycogen-Pellet am Boden des Reaktionsgefäßes 4 entsteht.
16. Isopropanol verwerfen, das DNA-Glycogen-Pellet verbleibt im Reaktionsgefäß 4.
17. Zum DNA-Glycogen-Pellet 600 µl 70 % Ethanol pipettieren und das Reaktionsgefäß 4 20-mal invertieren.
18. Das Reaktionsgefäß 4 1 min bei 16.000 x g zentrifugieren.
19. 70 % Ethanol verwerfen (mit Pipette entfernen), das DNA-Glycogen-Pellet verbleibt im Reaktionsgefäß 4.
20. Das DNA-Glycogen-Pellet im geöffneten Reaktionsgefäß 25 entweder 10 min bei 37 °C im verschlossenen Thermoblock oder 15 bis 20 min unter der Sterilwerkbank trocknen.
21. Pellet in 50 bis 100 µl Rehydratisierungspuffer (10 mM Tris, 1 mM EDTA, pH 7-8, DNA- und DNase-frei) 1 h lösen bei 65 °C im Thermoblock (im geschlossenen Reaktionsgefäß).
22. Analyse/Detektion mit quantitativer Realtime-PCR, z.B. mit universellen oder spezifischen bakteriellen Primern.

Reaktionsbedingungen Beispiel 1:
25-µl-PCR-Reaktionsvolumen (12,5 µl "MAXIMA SYBR Green qPCR Master Mix" von Fermentas, 10 nM ROX,
   0,3 µM Forward Primer SEQ ID NO. 1:
   5'-AAGTCGAGCGGACAGATGG-3',
   0,3 µM Reverse Primer SEQ ID NO. 2:
   5'-TGCGGTTCAAACAACCATCCG-3',
10 µl DNA (nach bevorzugter Ausführungsform der Erfindung gewonnen),
   mit Wasser ("PCR-Grade") auf 25 µl auffüllen.
   Temperaturprofil: 10 min 95 °C; 40 Zyklen mit 15 Sekunden 95 °C, 30 Sekunden 60 °C, 30 Sekunden 72 °C (Fluoreszenzdetektion bei 72°C); Schmelzkurve mit 1 min bei 95 °C, 30 Sekunden bei 55 °C, Temperaturrampe bis auf 95 °C mit Fluoreszenzmessung, 30 Sekunden 95 °C.

Ergebnisse des Ausführungsbeispiels 1:

**Tabelle 1: Ct-Werte ("Cycle Threshold", Schwellenwert-Zyklus) und Schmelzpunkte von Ausführungsbeispiel 1**

| Probenbezeichnung | Cycle Threshold | Schmelztemperatur des Amplikons [°C] |
|---|---|---|
| 2x10² CFU/ml* | 34,22 | 83,80 |
| 2x10² CFU/ml* | 34,37 | 83,80 |
| 2x10³ CFU/ml* | 33,48 | 83, 80 |
| 2x10³ CFU/ml* | 33,90 | 83,80 |
| 2x10⁴ CFU/ml* | 32,18 | 83, 80 |
| 2x10⁴ CFU/ml* | 32,21 | 83,80 |
| 2x10⁵ CFU/ml* | 29,05 | 83,80 |
| 2x10⁵ CFU/ml* | 28,91 | 83,80 |
| PCR-Negativkontrolle | No Ct | 69,38 |
| PCR-Negativkontrolle | No Ct | 69,38 |
| PCR-Negativkontrolle | No Ct | 69,38 |

| | | |
|---|---|---|
| *Durch Ausplattieren ermittelte CFU-Konzentrationen (CFU = Colony-forming unit). | | |

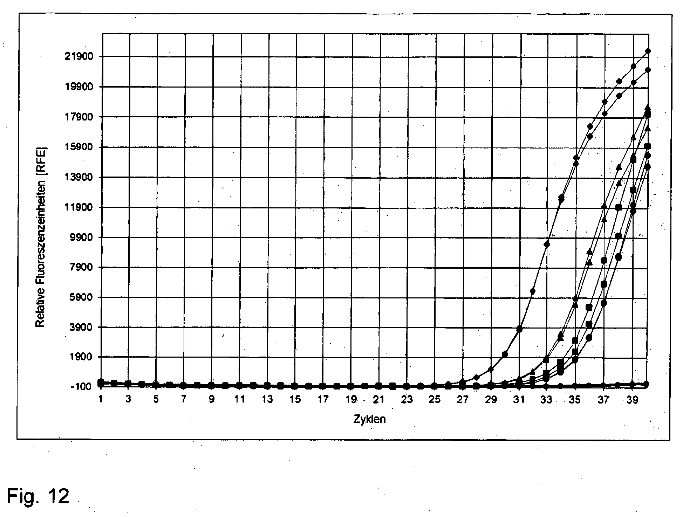

Figur 12 zeigt die Kurvenverläufe der nach der bevorzugten Ausführungsform der Erfindung prozessierten *B. subtilis* Proben: Rhomben (2x10⁵ CFU/ml), Dreiecke (2x10⁴ CFU/ml), Quadrate (2x10³ CFU/ml), Kreise (2x10² CFU/ml), Sterne (0 CFU/ml, Extraktions-Negativkontrollen). Unter Verwendung der bevorzugten Ausführungsform der Erfindung kann in einem beliebig großen Probevolumen *Bacillus subtilis* mindestens mit einer Sensitivität von 2x10² CFU/ml nachgewiesen werden (unter Verwendung der Primer mit den Sequenzen SEQ 1 und SEQ 2).

### Beispiel 2

Erfindung mit bevorzugter Ausführungsform vs. Stand der Technik (L. J. DiMichele, Am. Soc. Brew. Chem., 1993, vol.51 No.2, S. 63-66 und K. Stärk, Applied and Environmental Microbiology, 1998, Vol.64, No.2, S. 543-548; Weiterbehandlung des gelösten Filtrationsmediums 37 ohne Zelllyseschritt). Sensitivitätsvergleich für die Detektion von *B. subtilis* Sporen unter Verwendung der Vorrichtungen 1 inkl. Auffanggefäß 4 und Filtrationsvorrichtung 32.

Zwei Membranfilter/Filtrationsmedium 37 wurden nach einer bevorzugten Ausführungsform der Erfindung prozessiert (d.h. Zellaufschluss mithilfe von Mahlkugeln 29 in einem Homogenisator). Zwei Membranfilter/Filtrationsmedium 37 wurden nach dieser bevorzugten Ausführungsform der Erfindung prozessiert, jedoch ohne Zelllyseschritt (entspricht Stand der Technik nach K. Stärk und L. J. DiMichele). Zwei Membranfilter/Filtrationsmedium 37 wurden als Extraktions-Negativkontrollen nach der bevorzugten Ausführungsform der Erfindung prozessiert, jedoch ohne Beaufschlagung von Mikroorganismen. Jeder Membranfilter/Filtrationsmedium 37 wurde mit 10⁶ *B. subtilis* Sporen beaufschlagt bzw. die zwei Extraktions-Negativkontrollen wurden nur mit sterilem Wasser (PCR-Grade) in Kontakt gebracht. Die sechs Proben wurden analog wie in Beispiel 1 beschrieben nach der bevorzugten Ausführungsform der Erfindung prozessiert (Schritt 1 bis 22). Bei den Proben nach dem Stand der Technik (nach K. Stärk und L. J. DiMichele) wurden Auffanggefäße 4 ohne Mahlkugeln 29 verwendet und der Zelllyseschritt im Homogenisator wurde ausgelassen.

Ergebnisse des Ausführungsbeispiels 2:

**Tabelle 2: Ct-Werte und Schmelzpunkte von Ausführungsbeispiel 2**

| Probenbezeichnung | Cycle Threshold | Schmelztemperatur des Amplikons [°C] |
|---|---|---|
| PCR-Negativkontrolle | No Ct | 56,92 |
| PCR-Negativkontrolle | No Ct | 56, 91 |
| Extraktions-Negativkontrolle | No Ct | 56,91 |
| Extraktions-Negativkontrolle | No Ct | 56,46 |
| Stand der Technik | 33,23 | 83,97 |
| Stand der Technik | 33, 55 | 83,97 |
| Erfindung (bevorzugte Ausführungsform) | 29,96 | 83,97 |
| Erfindung (bevorzugte Ausführungsform) | 29,86 | 83,97 |

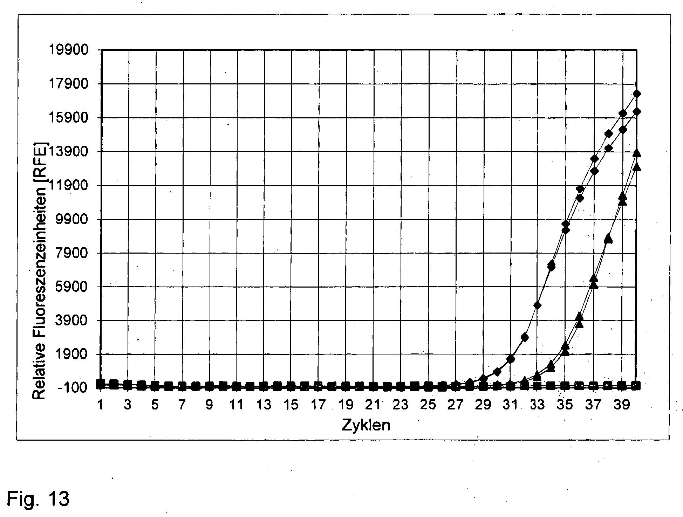

Figur 13 zeigt die Kurvenverläufe der nach der bevorzugten Ausführungsform der Erfindung prozessierten *B*. *subtilis* Sporen-Proben im Vergleich zum Stand der Technik: Rhomben (Erfindung mit bevorzugter Ausführung), Dreiecke (Stand der Technik), Quadrate (PCR-Negativkontrollen und Extraktions-Negativkontrollen).

Beispiel 2 zeigt, dass die Erfindung in der bevorzugten Ausführungsform dem Stand der Technik überlegen ist, da eine Sensitivitätssteigerung um mehr als 3 Ct-Einheiten erreicht werden konnte, was einem Faktor von etwa zehn Genomeinheiten/*B*. *subtilis* Sporen entspricht.

### Bezugszeichenliste

- 1: Vorrichtung
- 2: Aufnahmeeinheit
- 3: Auslass von 2
- 4: Auffanggefäß
- 5: Aufnahmeteil von 2
- 6: Bodenteil von 2
- 7: Außenwandung von 5
- 8: Innenwandung von 5
- 9: Deckenwandung
- 10: Aufnahmeteilinnenfläche
- 11: Fixierrand
- 12: Haftschicht
- 13: innere Außenwandungsfläche
- 14: Wulst von 5
- 15: Außenwandung von 6
- 16: Außenfläche von 15
- 17: Inkubationsraum
- 18: Abflussfläche
- 20: Winkel
- 21: Auslasskanal
- 22: Langsachse von 6
- 23: lichte Weite von 21
- 24: Absatz von 19
- 26: offenes Ende von 25
- 27: Außengewinde von 25
- 28: Lösungsmittel
- 29: Mahlkugeln
- 30: Vertiefung von 6
- 31, 31': Verrastung
- 32: Filtrationsvorrichtung
- 33: Unterteil von 32
- 34: Aufnahmeersatz von 33
- 35: Aufsatz
- 36: Filterunterstützungsfläche
- 37 -: Filtrationsmedium
- 38: Rand von 37
- 39: Zentrifugenadapter
- 40: Aussparung

## Patentansprüche

1. Vorrichtung (1) zur Behandlung eines porösen Filtrationsmediums (37) mit einer aus einem Aufnahmeteil (5) und einem Bodenteil (6) bestehenden Aufnahmeeinheit (2), wobei mit dem Aufnahmeteil (5) das poröse Filtrationsmedium (37) von einem Unterteil (33) einer Filtrationsvorrichtung (32) aufnehmbar und das Aufnahmeteil (5) mit dem porösen Filtrationsmedium (37) auf das Bodenteil (6) aufsetzbar ist, und wobei das Aufnahmeteil (5) mit dem Bodenteil (6) verrastbar ausgebildet ist, wobei das Bodenteil (6) zum Filtrationsmedium (37) hin einen Inkubationsraum (17) aufweist, der mit einem dem Aufnahmeteil (5) abgewandten Auslass (3) des Bodenteils (6) verbunden ist,
**dadurch gekennzeichnet,**
**dass** der Inkubationsraum (17) des Bodenteiles (6) zum Auslass (3) hin konisch verläuft,
**dass** der Auslass (3) einen Ansatz (24) aufweist, auf den ein ein Lösungsmittel (28) zum Auflösen des porösen Filtrationsmediums (37) enthaltendes Auffanggefäß (4) lösbar aufsteckbar ist,
**dass** das auf das Bodenteil (6) aufzusteckende Auffanggefäß (4) neben dem Lösungsmittel (28) den Zellaufschluss unterstützende Mahlkugeln (29) enthält, und
**dass** der Auslass (3) des Bodenteils (6) einen Auslasskanal (21) aufweist, der quer zur Längsachse (22) des Bodenteils (6) als länglicher Schlitz ausgebildet ist, dessen schmale lichte Weite (23) kleiner ist als die Außendurchmesser der Mahlkugeln (29).

2. Vorrichtung nach Anspruch 1,
**dadurch gekennzeichnet,**
**dass** das Lösungsmittel (28) zum Auflösen des Filtrationsmediums (37) ein organisches Lösungsmittel ist.

3. Vorrichtung nach einem der Ansprüche 1 oder 2,
**dadurch gekennzeichnet,**
**dass** das Lösungsmittel (28) Chloroform oder Methylenchlorid ist.

4. Vorrichtung nach einem der Ansprüche 1 bis 3,
**dadurch gekennzeichnet,**
**dass** das auf das Bodenteil (6) aufzusteckende Auffanggefäß (4) neben dem Lösungsmittel (28) einen den Zellaufschluss unterstützenden Lysepuffer enthält.

5. Vorrichtung nach einem der Ansprüche 1 bis 4,
**dadurch gekennzeichnet,**
**dass** das Auffanggefäß (4) an seinem offenen Ende (26) durch einen Deckel flüssigkeitsdicht verschließbar ist.

6. Vorrichtung nach einem der Ansprüche 1 bis 5,
**dadurch gekennzeichnet,**
**dass** eine Innenwandung (8) des Aufnahmeteils (5) außerhalb einer für eine Filtration nutzbaren Fläche des Filtrationsmediums (37) positionierbar ist und ein in dem Aufnahmeteil (5) angeordneter Fixierrand (11) auf einem Rand (38) des Filtrationsmediums (37) positionierbar ist, und
**dass** der Fixierrand (11) des Aufnahmeteils (5) mit dem Rand (38) des Filtrationsmediums (37) über eine Klebehaftung verbindbar ist.

7. Vorrichtung nach einem der Ansprüche 1 bis 6,
**dadurch gekennzeichnet,**
**dass** das Filtrationsmedium (37) aus Polycarbonat oder Polyethersulfon ausgebildet ist.

8. Vorrichtung nach einem der Ansprüche 1 bis 7,
**dadurch gekennzeichnet,**
**dass** die Aufnahmeeinheit (2) mit dem in vertikaler Richtung unten angeordneten Auffanggefäß (4) in einem Zentrifugenadapter (39) befestigbar ist und mit dem Zentrifugenadapter (39) in einer Zentrifuge zentrifugierbar ist, wobei das in dem Lösungsmittel (28) gelöste Filtrationsmedium (37) einschließlich zurückgehaltener Mikroorganismen vollständig in das Auffanggefäß (4) überführbar ist.

9. Verfahren zur Behandlung eines porösen Filtrationsmediums (37) mit einer aus einem Aufnahmeteil (5) und einem Bodenteil (6) bestehenden Aufnahmeeinheit, (2) einer Vorrichtung (1),
- bei dem das Aufnahmeteil (5) auf das in einem Unterteil (33) einer Filtrationsvorrichtung (32) angeordnete und einer flüssigen Probe ausgesetzte Filtrationsmedium (37) aufgesetzt wird, wobei ein in dem Aufnahmeteil (5) angeordneter Fixierrand (11) mit einem Rand (38) des Filtrationsmediums (37) verbunden wird,
- bei dem das Aufnahmeteil (5) mit dem verbundenen Filtrationsmedium (37) von dem Unterteil (33) abgehoben und auf das Bodenteil (6) aufgesetzt wird, wobei das Aufnahmeteil (5) und das Bodenteil (6) miteinander verrastet werden,
**dadurch gekennzeichnet,**
**dass** ein ein Lösungsmittel (28) zum Auflösen des porösen Filtrationsmediums (37) und den Zellaufschluss unterstützende Mahlkugeln (29) enthaltendes Auffanggefäß (4) mit einem am Bodenteil (6) angeordneten Auslass (3) lösbar verbunden wird, und
**dass** die Aufnahmeeinheit (2) mit dem Auffanggefäß (4) kopfüber geschüttelt wird, wobei das Lösungsmittel (28) über den Auslass (3) des Bodenteils (6) dem Filtrationsmedium (37) zugeführt wird und das Filtrationsmedium (37) auflöst, und
**dass** die Aufnahmeeinheit (2) mit dem in vertikaler Richtung unten angeordneten Auffanggefäß (4) in einem Zentrifugenadapter (39) befestigt und in einer Zentrifuge zentrifugiert wird, wobei das in dem Lösungsmittel (28) gelöste Filtrationsmedium (37) einschließlich zurückgehaltener Mikroorganismen vollständig in das Auffanggefäß (4) überführt wird.

10. Verfahren nach Anspruch 9,
**dadurch gekennzeichnet,**
**dass** das Auffanggefäß (4) von der Aufnahmeeinheit (2) entfernt und mit einem Deckel verschlossen wird.

11. Verfahren nach Anspruch 9,
**dadurch gekennzeichnet,**
**dass** das Auffanggefäß (4) von der Aufnahmeeinheit (2) entfernt und mit einem Deckel verschlossen wird, wobei vor dem Verschließen mit dem Deckel die Zugabe von einem den Zellaufschluss unterstützenden Lysepuffer in das Auffanggefäß erfolgt.

12. Verfahren nach Anspruch 10 oder 11,
**dadurch gekennzeichnet,**
**dass** das verschlossene Auffanggefäß (4) in einem Schüttelinkubator oder Homogenisator prozessiert wird, wobei ein Zellaufschluss der Mikroorganismen mithilfe der Mahlkugeln (29) und des Lysepuffers ermöglicht wird.

## Claims

1. A device (1) for treating a porous filtration medium (37) having a receiving unit (2) consisting of a receiving part (5) and a base part (6), wherein the porous filtration medium (37) can be received via the receiving part (5) by a lower part (33) of a filtration device (32), and the receiving part (5) with the porous filtration medium (37) can be mounted on the base part (6), and wherein the receiving part (5) is formed so as to be latchable to the base part (6), wherein the base part (6), towards the filtration medium (37) has an incubation chamber (17) which is connected to a base part (6) outlet (3) that is facing away from the receiving part (5),
**characterised in that**
the incubation chamber (17) of the base part (6) is conical towards the outlet (3),
the outlet (3) has a projection (24) onto which a receiving vessel (4) containing a solvent (28) for dissolving the porous filtration medium (37) can be detachably pushed on,
the receiving vessel (4) to be push-fitted to the base part (6) contains both the solvent (28) and the grinding balls (29) which support cell disruption,
and
**in that** the outlet (3) of the base part (6) has an outlet channel (21) which is designed as an oblong slot arranged at a right angle to the longitudinal axis (22) of the base part (6), and the clear width (23) of which is smaller than the outside diameter of the grinding balls (29).

2. The device according to Claim 1,
**characterised in that**
the solvent (28) for dissolving the filtration medium (37) is an organic solvent.

3. The device according to any of Claims 1 or 2,
**characterised in that**
the solvent (28) is chloroform or methylene chloride.

4. The device according to any of Claims 1 to 3,
**characterised in that**
the receiving vessel (4) to be push-fitted to the base part (6) contains both the solvent (28) and a lysis buffer which supports cell disruption.

5. The device according to any of Claims 1 to 4,
**characterised in that**
the open end (26) of the receiving vessel (4) can be sealed with a cover to prevent fluids from leaking.

6. The device according to any of Claims 1 to 5,
**characterised in that**
an inner wall (8) of the receiving part (5) can be positioned outside a surface of the filtration medium (37) that can be used for filtration, and that a fixing edge (11) arranged in the receiving part (5) can be positioned on an edge (38) of the filtration medium (37), and
that the fixing edge (11) of the receiving part (5) can be attached to the edge (38) of the filtration medium (37) by means of an adhesive bond.

7. The device according to any of Claims 1 to 6,
**characterised in that**
the filtration medium (37) is made of polycarbonate or polyethersulfone.

8. The device according to any of claims 1 to 7,
**characterised in that**
the receiving unit (2) can be attached to the receiving vessel (4) arranged vertically at the bottom in a centrifuge adapter (39) and be centrifuged with the centrifuge adapter (39)in a centrifuge, whereby the filtration medium (37) dissolved in the solvent (28), including retained microorganisms, can be completely transferred into the collection vessel (4).

9. Method for the treatment of a porous filtration medium (37) having a receiving unit (2), which consists of a receiving part (5) and a base part (6), of a device (1),
- in which the receiving part (5) is mounted on the filtration medium (37), which is arranged in a lower part (33) of a filtration device (32) and is exposed to a liquid sample, wherein a fixing edge (11) arranged in the receiving part (5) is connected with an edge (38) of the filtration medium (37),
- in which the receiving part (5) with the connected filtration medium (37) is lifted from the lower part (33) and placed on the base part (6), whereby the receiving part (5) and the base part (6) are latched together,
**characterised in that**
a receiving vessel (4) containing a solvent (28) for dissolving the porous filtration medium (37) and grinding balls (29) which support cell disruption is detachably connected to an outlet (3) arranged on the base part (6), and
that the receiving unit (2) with the receiving vessel (4) is inverted and shaken, whereby the solvent (28) is added to the filtration medium (37) via the outlet (3) of the base part (6), and dissolves the filtration medium (37), and
the receiving unit (2) is attached to the receiving vessel (4) arranged vertically at the bottom in a centrifuge adapter (39) and centrifuged in a centrifuge, whereby the filtration medium (37) dissolved in the solvent (28), including retained microorganisms, is completely transferred into the receiving vessel (4).

10. The method according to Claim 9,
**characterised in that**
the receiving vessel (4) is removed from the receiving unit (2) and sealed by a cover.

11. The method according to Claim 9,
**characterised in that**
the receiving vessel (4) is removed from the receiving unit (2) and sealed with a cover, with a lysis buffer to promote cell disruption being added to the receiving vessel before it is sealed with the cover.

12. The method according to Claim 10 or 11,
**characterised in that**
the sealed receiving vessel (4) is processed in an agitation incubator or homogenizer, with cell disruption of the microorganisms being facilitated by the grinding balls (29) and the lysis buffer.

## Revendications

1. Dispositif (1) de traitement d'un milieu de filtration poreux (37), comprenant un ensemble réceptacle (2) constitué d'une partie réceptacle (5) et d'une partie de fond (6), le milieu de filtration poreux (37) pouvant être retiré d'une partie inférieure (33) d'un dispositif de filtration (32) au moyen de la partie réceptacle (5) et ladite partie réceptacle (5) contenant le milieu de filtration poreux (37) pouvant être posée sur la partie de fond (6), et la partie réceptacle (5) étant conçue pour s'encliqueter avec la partie de fond (6), ladite partie de fond (6) comportant en direction du milieu de filtration (37) une chambre d'incubation (17) reliée à une évacuation (3) ménagée dans la partie de fond (6) à l'opposé de la partie réceptacle (5)
**caractérisé en ce que**
la chambre d'incubation (17) de la partie de fond (6) est conique vers l'évacuation (3), l'évacuation (3) comporte un embout (24) sur lequel un récipient de collecte (4) contenant un solvant (28) servant à dissoudre le milieu de filtration poreux (37) peut être enfiché de manière amovible,
le vase réceptacle (4) à enficher sur la partie de fond (6) contient un solvant (28) et les billes de broyage (29) qui soutiennent la désintégration cellulaire,
et
que l'évacuation (3) de la partie de base (6) comporte un canal d'évacuation (21) ayant la forme d'une fente oblongue disposée à un angle droit par rapport à l'axe longitudinal (22) de la partie de base (6), et dont la largeur utile (23) est inférieure au diamètre extérieure des billes de broyage (29).

2. Dispositif selon la revendication 1, **caractérisé en ce que** le solvant (28) servant à dissoudre le milieu de filtration (37) est un solvant organique.

3. Dispositif selon l'une quelconque des revendications 1 ou 2, **caractérisé en ce que** le solvant (28) est le chloroforme ou le chlorure de méthylène.

4. Dispositif selon l'une quelconque des revendications 1 à 3, **caractérisé en ce que** le vase réceptacle (4) à enficher sur la partie de fond (6) contient à la fois le solvant (28) et un tampon de lyse qui soutient la désintégration cellulaire.

5. Dispositif selon l'une quelconque des revendications 1 à 4, **caractérisé en ce que** l'extrémité ouverte (26) du vase réceptacle (4) peut être fermée à l'aide d'un couvercle pour prévenir les fuites de fluides.

6. Dispositif selon l'une quelconque des revendications 1 à 5, **caractérisé en ce qu'**une paroi interne (8) de la partie réceptacle (5) peut être placée à l'extérieur d'une surface du milieu de filtration (37) utilisable pour la filtration, et qu'un bord de fixation (11) disposé dans la partie réceptacle (5) peut être placée sur un bord (38) du milieu de filtration (37), et
que le bord de fixation (11) de la partie réceptacle (5) peut être attaché au bord (38) du milieu de filtration (37) au moyen d'une liaison adhésive.

7. Dispositif selon l'une quelconque des revendications 1 à 6, **caractérisé en ce que** le milieu de filtration (37) est fait de polycarbonate ou de polyéthersulfone.

8. Dispositif selon l'une quelconque des revendications 1 à 7, **caractérisé en ce que** l'ensemble réceptacle (2) peut être fixé au vase réceptacle (4) disposé verticalement au fond d'un adaptateur de centrifugation (39) et peut être centrifugé avec l'adaptateur de centrifugation (39) dans un centrifuge, le milieu de filtration (37) dissout dans le solvant (28), avec les microorganismes retenus, pouvant être entièrement transféré dans le vase réceptacle (4).

9. Procédé de traitement d'un milieu de filtration poreux (37) comportant un ensemble réceptacle (2), constitué d'une partie réceptacle (5) et d'une partie de base (6), d'un dispositif (1),
- dans lequel la partie réceptacle (5) est posée sur le milieu de filtration (37), qui est disposé dans une partie inférieure (33) d'un dispositif de filtration (32) et exposé à un échantillon de liquide, un bord de fixation (11) disposé dans la partie réceptacle (5) étant relié à un bord (38) du milieu de filtration (37),
- dans lequel la partie réceptacle (5) avec le milieu de filtration (37) relié est surélevée par rapport à la partie inférieure (33) et placée sur la partie de fond (6), la partie réceptacle (5) et la partie de fond (6) s'encliquetant l'un avec l'autre,
**caractérisé en ce que**
un vase réceptacle (4) contenant un solvant (28) servant à dissoudre le milieu de filtration poreux (37) et les billes de broyage (29) qui prennent en charge la désintégration cellulaire est raccordé de manière amovible à une évacuation (3) disposée sur une partie de fond (6), et que l'ensemble réceptacle (2) avec le vase réceptacle (4) est inversé et secoué, le solvant (28) étant ajouté au milieu de filtration (37) via l'évacuation (3) de la partie de fond (6), dissolvant le milieu de filtration (37), et
que l'ensemble réceptacle (2) avec le vase réceptacle (4) disposé verticalement au fond est fixé dans un adaptateur de centrifugation (39) et centrifugé dans un centrifuge, le milieu de filtration (37) dissout dans le solvant (28), comprenant les microorganismes retenus, étant entièrement transféré dans le vase réceptacle (4).

10. Méthode selon la revendication 9, **caractérisé en ce que** le vase réceptacle (4) est enlevé de l'ensemble réceptacle (2) et fermé à l'aide d'un couvercle.

11. Méthode selon la revendication 9, **caractérisé en ce que** le vase réceptacle (4) est enlevé de l'ensemble réceptacle (2) et fermé à l'aide d'un couvercle, un tampon de lyse pour faciliter la désintégration cellulaire étant ajouté au vase réceptacle avant que ce dernier ne soit fermé à l'aide du couvercle.

12. Méthode selon la revendication 10 ou 11, **caractérisé en ce que** le vase réceptacle (4) fermé est traité dans un incubateur agitateur ou un homogénéisateur, la désintégration cellulaire des microorganismes étant facilitée par les billes de broyage (29) et le tampon de lyse.


### SEQUENCE LISTING

<110> Sartorius Stedim Biotech GmbH
<120> Vorrichtung und Verfahren zur Behandlung eines Filtrationsmediums
<130> SM1210-PCT
<150> DE102012015063.6
   <151> 2012-07-31
<160> 2
<170> BiSSAP 1.2
<210> 1
   <211> 19
   <212> DNA
   <213> Artificial Sequence
<220>
   <221> source
   <222> 1..19
   <223> /orgänism="Artificial Sequence" /note="Forward Primer" /mol_type="unassigned DNA"
<400> 1
   aagtcgagcg gacagatgg 19
<210> 2
   <211> 21
   <212> DNA
   <213> Artificial Sequence
<220>
   <221> source
   <222> 1..21
   <223> /organism="Artificial Sequence" /note="Reverse Primer" /mol_type="unassigned DNA"
<400> 2
   tgcggttcaa acaaccatcc g 21